# EUROPEAN PATENT SPECIFICATION

(11) **EP 0 495 504 B1**
(45) Date of publication and mention of the grant of the patent: **09.04.1997**
(21) Application number: 92100690.4
(22) Date of filing: 16.01.1992
(51) Int. Cl.: C07C 57/05, C07C 51/215

(54) **Process for production of methacrylic acid by catalytic oxidation of isobutane**
Verfahren zur Herstellung von Methacrylsäure durch katalytische Oxydierung von Isobutan
Procédé pour la fabrication de l'acide méthacrylique par l'oxydation catalytique d'isobutane

(30) Priority: 17.01.1991 JP 3647/91
(43) Date of publication of application: 22.07.1992
(73) Proprietor: SUMITOMO CHEMICAL COMPANY LIMITED, Osaka-shi, Osaka 541 (JP)
(72) Inventor: Nagai, Koichi, Niihama-shi (JP); Nagaoka, Yoshihiko, Niihama-shi (JP); Ishii, Norio, Niihama-shi (JP)
(74) Representative: Henkel, Feiler, Hänzel & Partner

(56) References cited:
- EP-A- 0 425 666
- US-A- 4 260 822
- US-A- 4 535 188
- PATENT ABSTRACTS OF JAPAN, vol. 11 no. 361 (C-459) [2808], 25 November 1987 & JP-A-62 132832

## Description

The present invention relates to a process for producing methacrylic acid by the oxidation of isobutane. More particularly, the present invention relates to a process for producing methacrylic acid by the catalytic oxidation of isobutane with molecular oxygen in a gas phase.

### DESCRIPTION OF RELATED ART

The process for producing methacrylic acid by two-step oxidation of isobutylene or tertiary butanol via methacrolein, is well known and is actually employed in industry. However, no process for producing methacrylic acid directly from isobutane, a less expensive material, has yet seen industrial application. JP-A-58-18913 discloses a process for producing methacrylic acid from isobutane, i.e. a process for producing methacrolein and methacrylic acid by dehydrogenating isobutane to convert it into isobutylene and, without effecting any separation and purification step, oxidizing isobutylene using a conventional catalyst for isobutylene oxidation. This process, however, requires a dehydrogenation step and is complex, and accordingly is not economical.

Meanwhile, processes are known for producing methacrylic acid and methacrolein directly from isobutane without conducting dehydrogenation of isobutane, by the oxidation of isobutane with a heteropoly-acid type catalyst. JP-A-55-62041 discloses a process for producing methacrylic acid by catalytically oxidizing isobutane directly with molecular oxygen in a gas phase in the presence of a heteropoly-acid type catalyst containing molybdenum, phosphorus and antimony.

JP-A-62-132832 discloses a process for producing methacrylic acid and/or methacrolein by allowing isobutane and oxygen to contact with a heteropoly-acid type catalyst alternately. JP-A-63-145249 discloses a process using a reduced heteropoly-acid catalyst.

JP-A-2-42032, JP-A-2-42033 and JP-A-2-42034 disclose processes each using a heteropoly-acid type catalyst containing phosphorus and/or arsenic as center element(s), molybdenum as a coordinated element and vanadium, copper or the like as essential elements.

The present inventors have previously filed an application for patent (JP-A-3-106839) on a heteropoly-acid type catalyst of particular composition having a higher activity, a longer life and better reaction selectivity than before.

Thus, it was found that the use of an appropriate catalyst enables production of methacrylic acid and methacrolein directly from isobutane at a relatively high selectivity although the partial oxidation of isobutane which is inactive had been thought to be difficult.

In the techniques disclosed in the above patent applications, however, the conversion of isobutane is very low (not higher than ten %) and, in terms of per-pass yield, 6-7% at best. The conversion is expected to become higher with the improvement of the catalyst used; even in that case, however, the recycling of unreacted isobutane in reactor is presumed to be necessary.

The recycling of unreacted isobutane can be conducted by subjecting the gas after the separation of condensible component (e.g. methacrylic acid, methacrolein) to absorption by an appropriate liquid (e.g. C₈₋₁₀ paraffinic oil) and subsequent stripping, as disclosed in JP-A-58-189130. Also in JP-A-2-256625 is disclosed a similar recycling method in which unreacted isobutane is absorbed by an organic solvent and separated.

Further, JP-A-2-4753 discloses a method for recovering unreacted alkane, etc. by pressure swing adsorption (PSA), in the production of nitrile or oxide from alkane.

The conventional method of allowing an organic solvent to absorb unreacted isobutane for separation thereof, is inevitably employed when there is used, as the source for molecular oxygen, air or oxygen-enriched air which contains a large amount of an inert gas. When the conversion of isobutane is low, however, the method must be able to recover a large amount of unreacted isobutane at a recovery ratio close to 100%, making large the apparatus and energy used for recovery.

The PSA method has a difficulty in achieving a sufficiently high recovery ratio of alkane, wasting a large amount of unreacted alkane.

The object of the present invention is to provide an economical recycling process which can be adopted even when the per-pass conversion of isobutane is low.

The present inventors made extensive study in order to develop an economical process for synthesis of methacrylic acid. As a result, it was found that the above object can be achieved by a process comprising a step of converting the carbon monoxide formed by the reaction, into carbon dioxide and a step of removing carbon dioxide by absorption. The finding has led to the completion of the present invention.

### SUMMARY OF THE INVENTION

According to the present invention, there is provided a process for producing methacrylic acid, which comprises the steps of:
(A) subjecting isobutane to gas phase oxidation with molecular oxygen in the presence of a solid catalyst to obtain a reaction product gas containing methacrylic acid, methacrolein, acetic acid, water, unreacted isobutane, oxygen, carbon monoxide and carbon dioxide,
(B) separating the reaction product gas into a condensible component containing methacrylic acid, methacrolein, acetic acid and water and a non-condensible gas component containing unreacted isobutane, oxygen, carbon monoxide and carbon dioxide,
(C) catalytically oxidizing the carbon monoxide in the non-condensible gas component, with oxygen to convert it into carbon dioxide,
(D) removing the carbon dioxide in the non-condensible gas component, and
(E) recycling the non-condensible gas component which has passed through the steps (C) and (D), in the step (A),
the order of carrying out the step (C) and the step (D) being reversible.

### BRIEF DESCRIPTION OF THE DRAWING

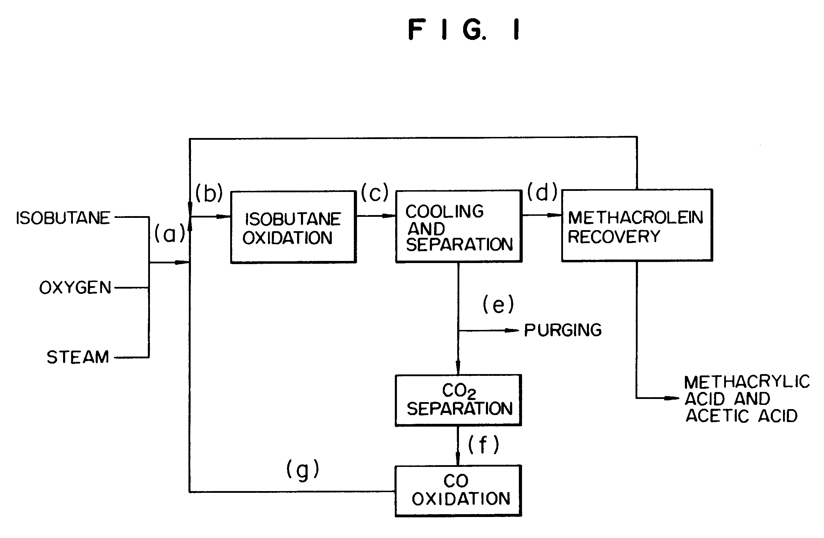

Fig. 1 is an illustration showing an example of the process of the present invention. In the drawing, (a), (b), (c), (d), (e), (f) and (g) indicate mixtures being transferred between the process steps. The specific compositions of the mixtures are shown in Table 1.

### DETAILED DESCRIPTION OF THE INVENTION

The solid catalyst used in the present invention used for direct oxidation of isobutane to produce methacrylic acid and methacrolein is not particularly restricted. However, solid catalysts each containing a heteropoly-acid and/or the salt thereof (the catalysts are hereinafter referred to as heteropoly-acid type catalysts) show the best performance for the present and accordingly, a heteropoly-acid type catalyst is generally used in the present invention. Heteropoly-acid type catalysts are represented by, for example, the general formula,

PₐMo_{b}X_{c}Y_{d}Zₑ

wherein P represents phosphorus; Mo represents molybdenum; X represents at least one element selected from the group consisting of rubidium, cesium and thallium; Y represents vanadium and/or arsenic; Z represents at least one element selected from the group consisting of copper, silver, bismuth, iron, cobalt, antimony, lanthanum and cerium; and a, b, c, d and e represent the atomic ratios of the individual elements with a proviso that when b = 12, a, c and d are each a value not larger than 3 but excluding 0 (zero) and e is a value not larger than 3 and including 0 (zero). Heteropoly-acid type catalysts consisting of element combinations such as Mo-P-V-Cs, Mo-P-V-Cs-Cu, Mo-P-As-Cs, Mo-P-As-V-Cs-Cu and the like exhibit excellent performances.

The conversion of isobutane is generally 10 % at best. When the isobutane concentration in material gas is low, the productivity of methacrylic acid is extremely low, making the industrial production infeasible. Therefore, the isobutane concentration in material gas is required to be generally 10-70%, preferably at least 20%.

Isobutane as a material is desired to have a high purity. It is because the components which are not reacted on the catalyst, are inevitably concentrated by recycling and it becomes necessary to increase the amount of the recycling gas partially purged. Propane, n-butane, etc. are less reactive than isobutane and, if they are reacted, it causes increase in impurities such as acrylic acid, maleic anhydride and the like. However, isobutylene may be present because isobutylene is reacted to produce methacrylic acid.

The molar ratio of oxygen/isobutane in material gas is usually 0.1-2, preferably 0.2-1. Although a larger molar ratio of oxygen gives a higher isobutane conversion, the molar ratio of oxygen has a restriction because attention must be paid to the inflammability limit of material gas. When the molar ratio of oxygen is too small, oxygen is insufficient for the oxidation of isobutane and carbon monoxide and, moreover, the catalyst is reduced excessively with the progress of reaction, which may invite deterioration in catalyst performance.

As the molecular oxygen source used in the present invention, there is used an oxygen of high purity, preferably an oxygen gas containing at least 97% by volume of oxygen. When the oxygen source is air or an oxygen-enriched air, a large amount of nitrogen is brought into the reaction system, making the process of the present invention substantially infeasible. In such a case, a process is more advantageously used wherein isobutane is selectively recovered according to a known method and recycled.

It is preferable that 5-30% of steam be present in the material gas. The presence of steam has an effect of increasing the isobutane conversion and the selectivities of methacrylic acid and methacrolein when a heteropoly-acid type catalyst is used.

The oxidation reaction of isobutane is usually conducted at 250-350°C and, in view of the catalyst life, preferably at temperatures not higher than 330°C. The reaction pressure can be selected in a wide range from reduced pressure to applied pressure, but generally in a range of from normal pressure to 5 atm. The contact time between the material gas and the catalyst is selected in a range of 1-10 seconds.

The reaction product gas obtained by the oxidation of isobutane with molecular oxygen contains, as a condensible component, water, methacrylic acid, methacrolein, acetic acid, very small amounts of high-boiling by-products, etc. and, as a non-condensible component, unreacted isobutane, oxygen and by-products such as carbon dioxide, carbon monoxide and the like. Substantially no lower hydrocarbons such as methane, ethane and the like are formed unlike the case of the dehydrogenation reaction of isobutane.

The reaction product gas is subjected to rapid cooling, absorption and the like to condense a condensible component. The condensate is sent to the subsequent separation and purification steps. The separated methacrolein, when recycled in the reactor, can be oxidized to methacrylic acid on the same catalyst. Alternatively, the methacrolein may be made into a product as it is, or may be transferred to an independent reactor for methacrolein oxidation to convert into methacrylic acid.

The residual aqueous methacrylic acid solution is subjected to extraction, distillation or the like to remove water, and the resulting material is purified as it is or after being esterified with methanol, to obtain a product, i.e. methacrylic acid or methyl methacrylate. If desired, acetic acid as a by-product can also be recovered as it is or in the form of an ester.

The non-condensible gas containing unreacted isobutane and oxygen is circulated to the reactor. However, since the gas further contains the carbon dioxide and carbon monoxide generated in the reaction, in fairly large amounts and cannot be recycled as it is, the carbon dioxide and carbon monoxide are removed. The biggest feature of the present invention is to remove the carbon monoxide and carbon dioxide formed by the reaction and being the major components of the non-condensible gas, efficiently out of the system.

The separation of carbon dioxide and carbon monoxide from the circulation gas can be realized by having a step for carbon dioxide separation and a step for carbon monoxide separation, independently. This, however, requires a very large facility and a large energy, which is not advantageous.

The presence itself of carbon monoxide and carbon dioxide in material gas has no problem. Accordingly, it should be sufficient to have only a step for absorption and stripping of carbon dioxide, if carbon monoxide is converted into carbon dioxide in the main reactor. However, it was found that carbon monoxide is not substantially reacted on the heteropoly-acid type catalyst generally used for production of methacrylic acid from isobutane at a high selectivity, if the reaction conditions in the main reactor are those suitable for the oxidation of isobutane.

This problem has been solved in the present invention by including, in the recycling system, a step for oxidizing carbon monoxide selectively to convert it into carbon dioxide.

The present inventors found that a known catalyst for low temperature oxidation of carbon monoxide can be used to selectively oxidize carbon monoxide with no oxidation of isobutane.

Such a catalyst includes, for example, a catalyst comprising a carrier such as alumina, silica or the like and a noble metal such as palladium, platinum or the like, supported on the carrier; a catalyst which is gold supported on a metal oxide such as iron oxide, cobalt oxide or the like in a high dispersion state; and a base metal compound oxide catalyst containing manganese as a main component, such as hopcalite catalyst or the like.

The conversion of carbon monoxide into carbon dioxide is conducted by passing a recycling gas through a layer of the above-mentioned catalyst at a temperature ranging from room temperature to not higher than 200°C at a contact time of 0.05-0.5 second. When the temperature is higher than 200°C, even isobutane is oxidized, which is not preferable. The heat of reaction causes rise in gas temperature; therefore, it is preferable to use a catalyst with which the difference in oxidation-starting temperatures of carbon monoxide and isobutane is as large as possible. Further, there is used, as necessary, such a reactor as to enable removal of heat of reaction, for example, a multi-tubular reactor.

The step of conversion of carbon monoxide into carbon dioxide is desirably conducted before or after the step for separation of carbon dioxide in the circulating gas, conducted after the separation of the condensible component containing methacrylic acid, methacrolein, etc. It is considered to effect the step at the outlet of the reactor or in its vicinity, but it is not desirable because the useful components such as methacrolein and the like are more combustible on the oxidation catalyst for carbon monoxide than isobutane.

As the method for separation of carbon dioxide, there are known methods such as absorption by liquid, adsorption and the like. Of them, most widespread and effected on a large scale is a method using an absorbing liquid containing hot potassium carbonate, ethanolamine or the like.

In the present invention, the method for separation of carbon dioxide is not particularly restricted, but there is used, for example, the known hot potassium carbonate method (the Benfield's method, the Catacarb method). The recycling gas is contacted with a solution containing potassium carbonate as a main component and a small amount of an amine (e.g. ethanolamine), in a counter current in an absorbing tower at 2-30 atm., whereby the carbon dioxide in the recycle gas is absorbed by the solution. The resulting solution is returned to around normal pressure in a regenerating tower; and the carbon dioxide in the solution is stripped by heat, whereby an absorbing solution is regenerated. Both absorption and regeneration are conducted at 100-130°C.

Neither conversion of carbon monoxide to carbon dioxide nor carbon dioxide removal by absorption needs to be 100%. Further, the conversion and removal may be conducted only on part of the recycling gas.

In the recycling process of the present invention wherein only carbon dioxide is separated, the material gas naturally contains diluting components other than isobutane, oxygen and steam. The other hydrocarbons and inorganic gases present in material isobutane gas, the impurities (e.g. argon, nitrogen) present in material oxygen, and the non-condensible gases generated by the reaction, other than carbon monoxide and carbon dioxide, are concentrated in the material gas by recycling, even when their amounts are very small. Carbon monoxide and carbon dioxide are concentrated according to their removal ratios.

In order to maintain the isobutane concentration at a desired level, it is necessary to purge the reaction gas out of the system by a small amount corresponding to the amount of the impurities which cannot be removed by absorption operation. The purged gas contains flammable gases such as high concentration isobutane and the like and accordingly can be effectively utilized by, for example, combusting it and recovering the generated heat.

An example of the process of the present invention is shown in Fig. 1. Isobutane, oxygen, steam, a recycling gas containing isobutane, etc., and a recovered methacrolein gas are fed into a step for isobutane oxidation. The reaction product gas is separated into a condensate and a non-condensible gas in a step for cooling and separation. The condensate is subjected to methacrolein separation in a step for methacrolein recovery, and the recovered methacrolein is fed into the step for isobutane oxidation. The residual methacrylic acid and acetic acid are purified or esterified to obtain products.

The non-condensible gas containing isobutane is partially purged as necessary in order to prevent the accumulation of inert gas. The non-condensible gas is subjected to carbon dioxide separation in a step for carbon dioxide separation and then to carbon monoxide oxidation in a step for carbon monoxide oxidation, and reused in the step for isobutane oxidation.

An example of the material balance in the process shown in Fig. 1 is shown in Table 1.

The yields of the isobutane reaction are such that methacrylic acid is 4.62%, methacrolein is 1.24%, carbon monoxide is 0.88%, carbon dioxide is 1.72% and acetic acid is 0.74%. The yields of the methacrolein oxidation are such that methacrylic acid is 64.0%, carbon monoxide is 8.0%, carbon dioxide is 10.0% and acetic acid is 8.0%. The removal ratio of carbon dioxide is 50%. The conversion in the carbon monoxide reactor is 71%. The material isobutane and the material oxygen each contain 0.5% of an inert component. The amount of the purged gas is 1% of the recycling gas.

In a process in which only isobutane is recovered and recycled, an isobutane recovery ratio close to 100% is required, making large both the apparatus for absorption and stripping step and the required energy. In contrast, the process of the present invention can be effected using a small facility and a small energy.

The present invention is hereinafter described in detail by way of Example. However, the present invention is in no way restricted to the Example.

### Example 1

In 500 ml of ion-exchanged water were suspended 105.9 g of ammonium molybdate [(NH₄)₆Mo₇O₂₄·4H₂O], 1.82 g of vanadium pentoxide and 2.17 g of cupric phosphate [Cu₃(PO₄)₂·3H₂O]. Thereto was added 180 ml of a solution of 7.49 g of 85% phosphoric acid, 4.73 g of a 60% aqueous arsenic acid solution and 17.5 g of cesium nitrate in ion-exchanged water. The resulting slurry was refluxed at about 100°C for 15 hours with stirring.

The resulting mixture was placed in a stainless steel vat and heated at 150°C in an electric furnace to evaporate water. The residue was calcined at 250°C in the air and then at 435°C for 5 hours in a nitrogen stream. To the resulting powder was added 2% of graphite. The mixture was molded into cylinders of 5 mm in diameter and 5 mm in height to obtain a catalyst. The catalyst had a composition excluding oxygen, hydrogen and nitrogen, of Mo₁₂P_{1.5}As_{0.4}V_{0.4}Cu_{0.3}Cs_{1.8}.

9 Grams of the catalyst was filled into a Pyrex glass-made tubular reactor of 15 mm in inside diameter. Into the reactor was fed a material gas consisting of 42 mole % of isobutane, 33 mole % of oxygen, 12 mole % of steam and 13 mole % of nitrogen at a rate of 5,400 ml/h (reduced to normal state). The reaction pressure was 1.5 atm and the temperature of the reactor wall was 310°C.

After 15 hours, the reaction product gas was analyzed by gas chromatography. As a result, the isobutane conversion was 9.2%; the selectivity of methacrylic acid was 50.2%; the selectivity of methacrolein was 13.5%; the selectivity of acetic acid was 8.0%; the selectivity of carbon dioxide was 16.8%; and the selectivity of carbon monoxide was 9.5%. Besides, there were formed very small amounts of oxygen-containing compounds such as acetone, acrylic acid, maleic anhydride and the like.

The material isobutane contains about 0.5% in total of propane and n-butane. These components took no part in the reaction and accordingly showed no change. There was no formation of lower hydrocarbons such as methane and ethane. A very small amount (selectivity: 1.5%) of isobutylene (regarded to be an intermediate of reaction) was detected but this component can be partially converted into methacrylic acid by recycling.

The gas from the reactor outlet was subjected to absorption by water. The unabsorbed gas portion was introduced into a Pyrex glass-made reactor of 12 mm in inside diameter filled with 0.2 g of a 1% palladium-on-alumina catalyst (a product of N. E. Chemcat). The reaction temperature was controlled at 165°C. As a result, 99% of carbon monoxide was converted into carbon dioxide, but isobutane caused substantially no reaction. The reaction temperature was increased to 190°C and the oxidation of isobutane started.

Next, carbon dioxide is removed by allowing an aqueous potassium carbonate solution to absorb it. The unabsorbed gas portion containing isobutane is recovered in the oxidation reactor for isobutane.

Catalytic oxidation of isobutane was effected in the same manner as above except that the material gas fed into the oxidation reactor had a composition consisting of 42 mole % of isobutane, 33 mole % of oxygen, 12 mole % of steam, 8 mole % of carbon dioxide and 5 mole % of carbon monoxide. The results of the isobutane oxidation were substantially the same according to the calculation based on an assumption that the carbon monoxide and carbon dioxide in the material gas took no part in the reaction. Further, a reaction was conducted under the same conditions, using a material gas consisting of 5 mole % of carbon monoxide and the remainder of the air. The conversion of carbon monoxide was 0.7%.

A reaction was conducted under the same conditions as above except that the catalytic oxidation of carbon monoxide to convert into carbon dioxide was conducted at 160°C using 0.2 g of a hopcalite catalyst (a compound oxide of manganese and copper) in place of the supported palladium catalyst. 71% of carbon monoxide was converted. The run-away of the reaction temperature occurred from 187°C.

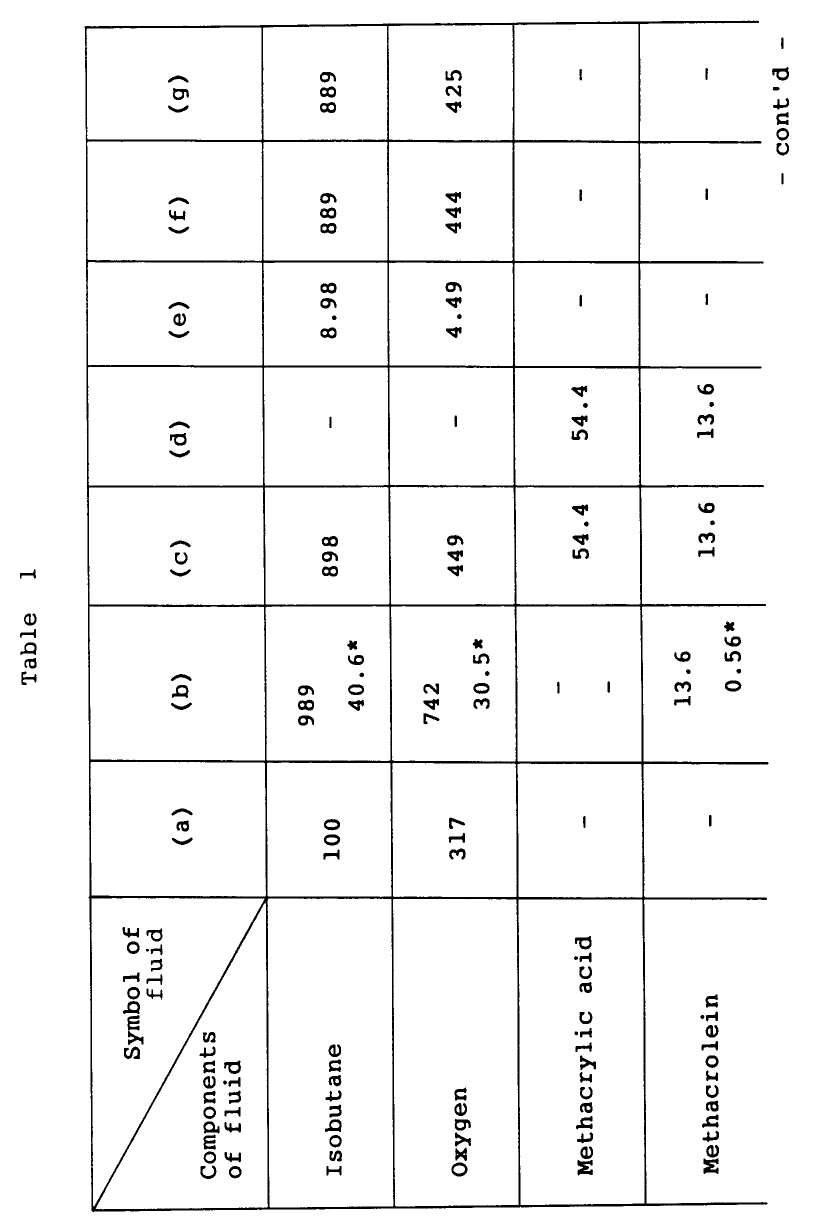

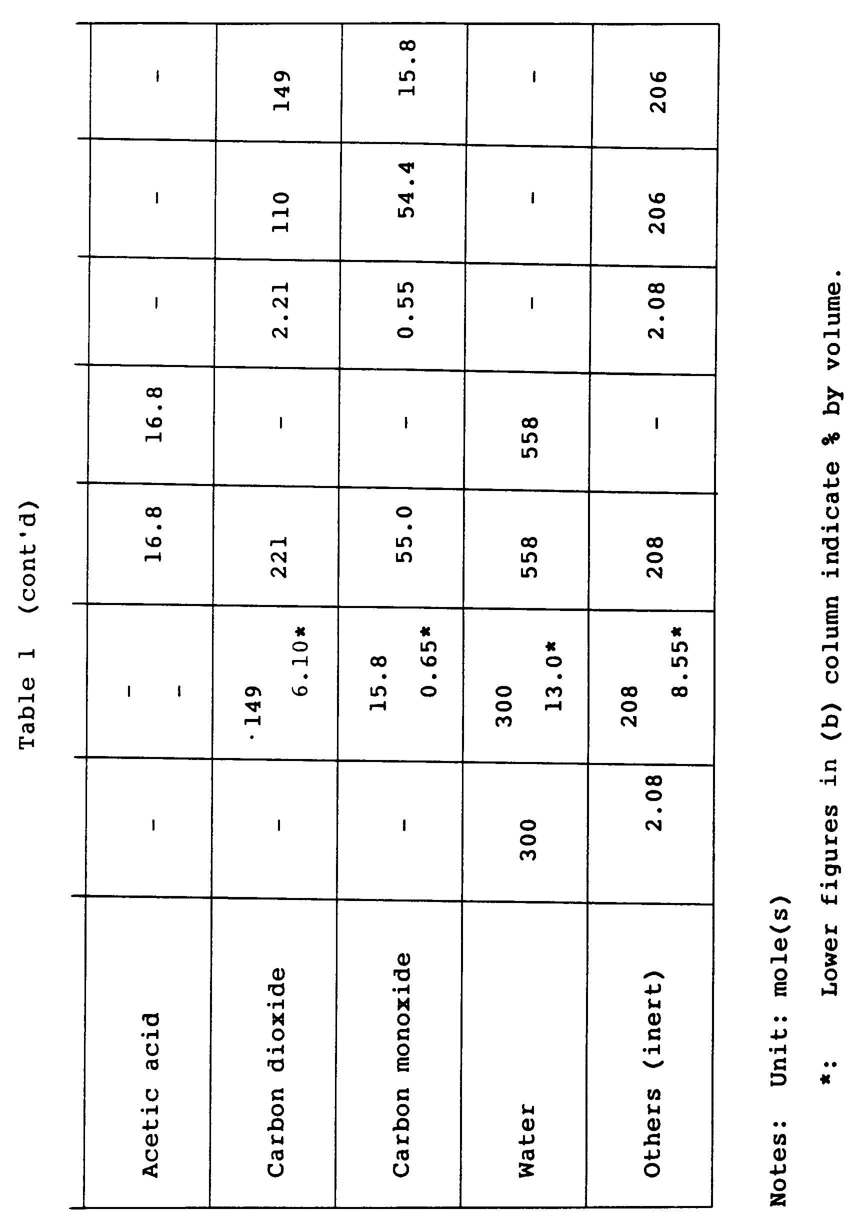

## Claims

1. A process for producing methacrylic acid, which comprises the steps of:
(A) subjecting isobutane to gas phase oxidation with molecular oxygen in the presence of a solid catalyst to obtain a reaction product gas containing methacrylic acid, methacrolein, acetic acid, water, unreacted isobutane, oxygen, carbon monoxide and carbon dioxide,
(B) separating the reaction product gas into a condensible component containing methacrylic acid, methacrolein, acetic acid and water and a non-condensible gas component containing unreacted isobutane, oxygen, carbon monoxide and carbon dioxide,
(C) catalytically oxidizing the carbon monoxide in the non-condensible gas component with oxygen to convert it into carbon dioxide,
(D) removing the carbon dioxide in the non-condensible gas component, and
(E) recycling the non-condensible gas component which has passed through the steps (C) and (D), in the step (A),
the order of carrying out the step (C) and the step (D) being reversible.

2. A process for producing methacrylic acid according to Claim 1, wherein the solid catalyst contains a heteropoly-acid and/or the salt thereof.

3. A process for producing methacrylic acid according to Claim 1, wherein the molecular oxygen source is a gas containing oxygen in an amount of at least 97% by volume.

4. A process for producing methacrylic acid according to Claim 1, wherein the catalyst used for catalytic oxidation of carbon monoxide with oxygen for conversion into carbon dioxide is a catalyst containing supported palladium and/or platinum, a catalyst containing supported gold or a catalyst containing manganese oxide.

5. A process for producing methacrylic acid according to Claim 1, wherein the removal of carbon dioxide is conducted by using an absorbent containing potassium carbonate as a main component.

6. A process for producing methacrylic acid according to Claim 1, wherein the solid catalyst is a heteropoly-acid represented by the general formula,
PₐMo_{b}X_{c}Y_{d}Zₑ
wherein P represents phosphorus; Mo represents molybdenum; X represents at least one element selected from the group consisting of rubidium, cesium and thallium; Y represents vanadium and/or arsenic; Z represents at least one element selected from the group consisting of copper, silver, bismuth, iron, cobalt, antimony, lanthanum and cerium; and a, b, c, d and e represent the atomic ratios of the individual elements with a proviso that when b = 12, a, c and d are each a value not larger than 3 but excluding 0 (zero) and e is a value not larger than 3 and including 0 (zero),
or the salt thereof.

7. A process for producing methacrylic acid according to Claim 1, wherein the molar ratio of molecular oxygen to isobutane in the oxidation reaction of isobutane is 0.1-2.

8. A process for producing methacrylic acid according to Claim 1, wherein the temperature of the oxidation reaction of isobutane is 250-350°C.

9. A process for producing methacrylic acid according to Claim 1, wherein the temperature of the oxidation reaction for conversion of carbon monoxide into carbon dioxide is room temperature to 200°C.

## Patentansprüche

1. Verfahren zur Herstellung von Methacrylsäure durch:
(A) Gasphasenoxidation von Isobutan mit molekularem Sauerstoff in Gegenwart eines festen Katalysators zur Gewinnung eines gasförmigen Reaktionsprodukts, das Methacrylsäure, Methacrolein, Essigsäure, Wasser, nicht umgesetztes Isobutan, Sauerstoff, Kohlenmonoxid und Kohlendioxid umfaßt,
(B) Auftrennen des gasförmigen Reaktionsprodukts in eine kondensierbare Komponente, die Methacrylsäure, Methacrolein, Essigsäure und Wasser enthält, und eine nicht kondensierbare gasförmige Komponente, die nicht umgesetztes Isobutan, Sauerstoff, Kohlenmonoxid und Kohlendioxid enthält,
(C) katalytische Oxidation des Kohlenmonoxids in der nicht kondensierbaren gasförmigen Komponente mit Sauerstoff zur Umwandlung desselben in Kohlendioxid,
(D) Entfernung des Kohlendioxids in der nicht kondensierbaren gasförmigen Komponente und
(E) Rückführen der nicht kondensierbaren gasförmigen Komponente, die die Stufen (C) und (D) durchlaufen hat, in Stufe (A),
wobei die Reihenfolge der Stufen (C) und (D) umkehrbar ist.

2. Verfahren zur Herstellung von Methacrylsäure nach Anspruch 1, wobei der feste Katalysator eine Heteropolysäure und/oder das Salz derselben enthält.

3. Verfahren zur Herstellung von Methacrylsäure nach Anspruch 1, wobei die Quelle für den molekularen Sauerstoff aus einem Sauerstoff in einer Menge von mindestens 97 Vol.-% enthaltenden Gas besteht.

4. Verfahren zur Herstellung von Methacrylsäure nach Anspruch 1, wobei der zur katalytischen Oxidation des Kohlenmonoxids mit Sauerstoff zur Umwandlung desselben in Kohlendioxd verwendete Katalysator aus einem Katalysator, der auf einem Träger befindliches Palladium und/oder Platin enthält, einem Katalysator, der auf einem Träger befindliches Gold enthält, oder einem Katalysator, der Manganoxid enthält, besteht.

5. Verfahren zur Herstellung von Methacrylsäure nach Anspruch 1, wobei die Entfernung von Kohlendioxid mit Hilfe eines Kaliumcarbonat als Hauptkomponente enthaltenden Absorptionsmittels erfolgt.

6. Verfahren zur Herstellung von Methacrylsäure nach Anspruch 1, wobei der feste Katalysator aus einer Heteropolysäure der allgemeinen Formel:
PₐMo_{b}X_{c}Y_{d}Zₑ
worin bedeuten:
P Phosphor;
Mo Molybdän;
X mindestens ein Element, ausgewählt aus der Gruppe Rubidium, Cäsium und Thallium;
Y Vanadium und/oder Arsen;
Z mindestens ein Element, ausgewählt aus der Gruppe Kupfer, Silber, Wismuth, Eisen, Kobalt, Antimon, Lanthan und Cer, und
a, b, c, d und e die Atomverhältnisse der einzelnen Elemente, wobei gilt, daß im Falle, daß b = 12 ist, a, c und d jeweils für eine Zahl von nicht mehr als 3 ausschließlich 0 (Null) stehen und e eine Zahl von nicht mehr als 3 einschließlich 0 (Null) bedeutet,
oder einem Salz derselben
besteht.

7. Verfahren zur Herstellung von Methacrylsäure nach Anspruch 1, wobei das Molverhältnis molekularer Sauerstoff/Isobutan bei der Oxidationsreaktion von Isobutan 0,1 bis 2 beträgt.

8. Verfahren zur Herstellung von Methacrylsäure nach Anspruch 1, wobei die Temperatur der Oxidationsreaktion von Isobutan 250-350°C beträgt.

9. Verfahren zur Herstellung von Methacrylsäure nach Anspruch 1, wobei die Temperatur der Oxidationsreaktion zur Umwandlung von Kohlenmonoxid in Kohlendioxid von Raumtemperatur bis 200°C reicht.

## Revendications

1. Procédé de production d'acide méthacrylique, comprenant les étapes consistant:
A) à soumettre de l'isobutane à une oxydation en phase gazeuse avec de l'oxygène moléculaire en présence d'un catalyseur solide, pour obtenir un produit de réaction gazeux contenant de l'acide méthacrylique, de la méthacroléine, de l'acide acétique, de l'eau, de l'isobutane n'ayant pas réagi, de l'oxygène, de l'oxyde de carbone et de l'anhydride carbonique,
B) à séparer le produit de réaction gazeux en un composant condensable qui contient de l'acide méthacrylique, de la méthacroléine, de l'acide acétique et de l'eau et en un composant gazeux non condensable qui contient l'isobutane n'ayant pas réagi, de l'oxygène, de l'oxyde de carbone et de l'anhydride carbonique,
C) à oxyder catalytiquement avec de l'oxygène l'oxyde de carbone contenu dans le composant gazeux non condensable, pour le convertir en anhydride carbonique,
D) à éliminer l'anhydride carbonique contenu dans le composant gazeux non condensable, et
E) à recycler dans l'étape A) le composant gazeux non condensable qui est passé par les étapes C) et D),
l'ordre d'exécution de l'étape C) et de l'étape D) étant réversible.

2. Procédé de production d'acide méthacrylique selon la revendication 1, dans lequel le catalyseur solide contient un hétéropolyacide et/ou un de ses sels.

3. Procédé de production d'acide méthacrylique selon la revendication 1, dans lequel la source d'oxygène moléculaire est un gaz contenant de l'oxygène dans une proportion d'au moins 97% en volume.

4. Procédé de production d'acide méthacrylique selon la revendication 1, dans lequel le catalyseur utilisé pour l'oxydation catalytique de l'oxyde de carbone par l'oxygène pour sa conversion en anhydride carbonique est un catalyseur contenant du palladium et/ou du platine sur support, un catalyseur contenant de l'or sur support ou un catalyseur contenant de l'oxyde de manganèse.

5. Procédé de production d'acide méthacrylique selon la revendication 1, dans lequel l'élimination de l'anhydride carbonique est effectuée au moyen d'un absorbant contenant du carbonate de potassium comme composant principal.

6. Procédé de production d'acide méthacrylique selon la revendication 1, dans lequel le catalyseur solide est un hétéropolyacide représenté par la formule générale
PₐMo_{b}X_{c}Y_{d}Zₑ
dans laquelle P représente un atome de phosphore, Mo représente un atome de molybdène, X représente au moins un élément choisi dans le groupe constitué par le rubidium, le césium et le thallium, Y représente un atome de vanadium et/ou d'arsenic, Z représente au moins un élément choisi dans le groupe constitué par le cuivre, l'argent, le bismuth, le fer, le cobalt, l'antimoine, le lanthane et le cérium, et a, b, c, d et e représentent les rapports atomiques des éléments individuels, étant spécifié que quand b = 12, a, c et d ont chacun une valeur non supérieure à 3, à l'exclusion de 0 (zéro) et e a une valeur non supérieure à 3, y compris 0 (zéro),
ou un de ses sels.

7. Procédé de production d'acide méthacrylique selon la revendication 1, dans lequel le rapport molaire de l'oxygène moléculaire à l'isobutane dans la réaction d'oxydation d'isobutane est de 0,1 à 2.

8. Procédé de production d'acide méthacrylique selon la revendication 1, dans lequel la température de la réaction d'oxydation d'isobutane est de 250 à 350°C.

9. Procédé de production d'acide méthacrylique selon la revendication 1, dans lequel la température de la réaction d'oxydation pour la conversion de l'oxyde de carbone en anhydride carbonique est comprise entre la température ambiante et 200°C.
